# EUROPEAN PATENT SPECIFICATION

(11) **EP 3 337 548 B1**
(45) Date of publication and mention of the grant of the patent: **29.09.2021**
(21) Application number: 16760257.2
(22) Date of filing: 18.08.2016
(51) Int. Cl.: A61M 25/02

(54) **NASOGASTRIC TUBE SECUREMENT SYSTEMS**
SYSTEME ZUR SICHERUNG EINES TRANSNASALEN MAGENBANDES
SYSTÈMES DE FIXATION D'UN TUBE NASOGASTRIQUE

(30) Priority: 21.08.2015 US 201562208055 P
(43) Date of publication of application: 27.06.2018
(73) Proprietor: 3M Innovative Properties Company, St. Paul, MN 55133-3427 (US)
(72) Inventor: OLIVEIRA, Jener de, CEP 13001-970 Sumaré (São Paulo) (BR); RAMIRES, Elaine C., 13084-230 Campinas (São Paulo) (BR); LOVON, Adriana S. P., CEP 13001-970 Sumaré (São Paulo) (BR); HEINECKE, Steven B., Saint Paul, Minnesota 55133-3427 (US); FUNG, Simon S., Saint Paul, Minnesota 55133-3427 (US); BIZARRIA, Felipe S. R., CEP 13001-970 Sumaré (São Paulo) (BR)
(74) Representative: Vossius & Partner Patentanwälte Rechtsanwälte mbB
(86) International application number: PCT/US2016/047489
(87) International publication number: WO 2017/034907

(56) References cited:
- WO-A1-94/28962
- WO-A1-2014/092703
- WO-A2-2012/009126
- US-A- 5 735 272
- US-A1- 2002 143 296
- US-A1- 2009 292 256
- US-A1- 2012 138 060

## Description

### FIELD

The present disclosure generally relates to nasogastric tube sccurcmcnt systems, and methods of using same, and particularly to systems configured to be secured to skin.

### BACKGROUND

During patient treatment (e.g., at hospitals, and particularly, in Intensive Care Units (ICUs)), the insertion of tubes can be required for different purposes, such as feeding, air supply, and/or liquid removal. The tubes inserted through the nose are referred to as nasogastric (NG) tubes and can be used for various applications, including feeding, drug administration and/or stomach drainage. Such nasogastric tubes generally need to be attached to the patient's skin in order to maintain the correct position internally, such as inside the stomach.

Some existing devices for NG tube securement do not allow a medical practitioner (e.g., a nurse) to intervene or evaluate the position or level of securement without removing an adhesive tape from skin, which can cause damage to the patient's skin (such as skin tears, redness, and/or damages due to constant changing of adhesives). Other existing devices arc large and bulky (e.g., configured to be attached around the patient's head), thereby being cumbersome to use, reducing patient comfort, and/or causing pressure ulcers.

US 2012/138060 A1 relates to a patient interface system comprising: a nasal seal of flexible material to communicate with at least one airway of the patient; and a primary headgear to which the nasal seal is removably attached, said primary headgear including a fabric and/or textile material connected to at least a portion of the nasal seal, with the primary headgear removably attachable to anadhesive strip positioned on the patient.

WO 2012/009126 relates to a nasal CPAP canula seal comprising: a canula supporting portion, defining apertures for canula passage into the nostrils; an anchoring portion designed to help anchor said nasal CPAP canula seal by adhering to facial areas about the nostrils; and wherein said anchoring portion defines a cut-out centered below said apertures, to help avoid covering any portion of the vermillion of the upper lip with said anchoring portion.

WO 2014/092703 relates to a strapless nasal interface device comprising: an interface body having a cavity therein, said cavity having a passageway to an air tube extending from said body, at least one nostril interface tube having an internal passage extending from said interface body, a facialpatch which is adherable to a portion of a person's face, and at least one attachment flap connected to said interface body and removably attachable to a surface of said facial patch adapted to hold said nostril interface tube in a position in which it communicates with a person's nostril when said facial patch is worn by such a person on the person's face and said attachment flap is attached to the surfaceof said facial patch.

WO 94/28962 A1 relates to a device for fixing a nasal tube in a person's one nostril, comprising an upper part for attachment by adhesion to the bridge of the person's nose, a lower part for attachmentby adhesion to the nasal tube, and an intermediate member which is contiguous with the upper part and the lower part, and which is arranged asymmetrically with respect to a vertical central axis of the upper part, characterized in that the device is symmetrical about a horizontal axis of symmetry.

US 5 735 272 A relates to a nasal tube holder for anchoring a tube in a nasal passage, comprising: a nasal dilator affixable over the bridge of a nose, the nasal dilator including a first end, a second end and biasing means for biasing said first end and said second end of the nasal dilator outwardly from the face of the user whereby the nasal passages are maintained in a dilated condition; a tube holding portion depending from the nasal dilator, for engaging a tube and anchoring the tube within a nasal passage; and an adhesive backing carried by the nasal dilator and the tube holding portion for adhering the nasal dilator to a nose and a tube to the tube holding portion.

US 2002/143296 A1 relates to a medical tube holding system comprising: a strip having first and second surfaces, the strip including adhesive on the first surface for attachment to a medical tube; and a tube holder including a first section for attachment to a patient, and a second section for attachment to the second surface of the adhesive strip.

US 2009/292256 A1 relates to a nasogastric tube Fastener comprising a fabric with a top side and a bottom side; the fabric having the shape of a top flap for attaching to a nose of a patient, a central strip that protrudes perpendicular from an edge of one end of the top flap; a plurality ofbottom flaps that protruding perpendicular from alternate edges of the central strip for attaching to a nasogastric tube, and a pressure s ensitive adhesive applied to the bottom side of the fabric.

### SUMMARY

The present invention is defined by the claims.

As a result, there is a need for robust, reliable, manipulatable, repositionable nasogastric tube securement systems, which provide for a standardization of procedures. The nasogastric tube securement systems of the present disclosure can increase the safety of NG tube securement and patient's comfort, while minimizing skin damage. Systems of the present disclosure generally allow for repositioning of the NG tube and/or the system (or a portion thereof) relative to a patient (e.g., the patient's skin, the nose, and/or an internal structure) when needed. In general, systems of the present disclosure include a base layer for securing the system to a patient's nose, and a coupling layer configured to be repositionably coupled to the base layer, while also being configured to secure the NG tube. The coupling layer can be repositioned without removing the base layer from the skin or even changing the base layer (e.g., comprising an adhesive tape) on the skin. In view of that, the comfort is enhanced and any potential risk for skin damage can be minimized.

The nasogastric tube securement systems of the present disclosure can also reduce pressure ulcers in the nostril caused by the NG tubes, which can be a frequent problem on patients using NG tubes. The majority of tubes are secured by tapes or adhesive devices that are usually changed after 24 hours which may increase the potential risk of pressure ulcers developing in the nostril. However, by using the nasogastric tube securement systems of the present disclosure, the medical practitioner can evaluate a potential pressure point inside the nostril and take action to avoid the ulcers by changing the securement device position without causing an adhesion lesion or reducing the securement of the NG tube.

Some aspects of the present disclosure provide a nasogastric tube securement system. The system includes a base layer and a coupling layer. The base layer is configured to be adhered to a nose. The base layer has a first major surface comprising a skin-contact adhesive and a second major surface opposite the first major surface, the base layer having a footprint area of A. The coupling layer includes a first end comprising coupling means configured to be repositionably coupled to the second major surface of the base layer, and the first end has a footprint area of at least 0.3A. The coupling layer includes a second end configured to secure a nasogastric tube, and a middle section connecting the first end and the second end. The middle section is elongated along a longitudinal direction, and the first end and the second end are each wider than the middle section in a lateral direction that is oriented substantially perpendicularly with respect to the longitudinal direction.

Other features and aspects of the present disclosure will become apparent by consideration of the detailed description and accompanying drawings.

### BRIEF DESCRIPTION OF THE DRAWINGS

FIG. 1 is a plan view of a nasogastric tube securement system according to one embodiment of the present disclosure, the system shown in the form of a kit, the system comprising a base layer and a coupling layer.
FIG. 2A is a side cross-sectional view of the base layer of the system of FIG. 1 according to one embodiment of the present disclosure, taken along line 2A-2A of FIG. 1.
FIG. 2B is a side cross-sectional view of the coupling layer of the system of FIG. 1 according to one embodiment of the present disclosure, taken along line 2B-2B of FIG. 1.
FIG. 3A is a side cross-sectional view of the base layer of the system of FIG. 1 according to another embodiment of the present disclosure.
FIG. 3B is a side cross-sectional view of the coupling layer of the system of FIG. 1 according to another embodiment of the present disclosure.
FIGS. 4A-4J illustrate a method of using the nasogastric tube securement system of FIG. 1.
FIGS. 5A and 5B each illustrate a base layer according to another embodiment of the present disclosure.
FIGS. 6A-6C each illustrate a coupling layer according to another embodiment of the present disclosure.
FIGS. 7A and 7B each illustrate a coupling layer according to another embodiment of the present disclosure.
FIGS. 8A and 8B illustrate a method of securing a nasogastric tube with the coupling layer of FIG. 7B.

### DETAILED DESCRIPTION

The present disclosure generally relates to nasogastric tube securement systems and methods of using same. Particularly, nasogastric tube securement systems of the present disclosure include at least two parts or components: (i) a base layer that can be coupled (i.e., adhered) to skin, and (ii) a coupling layer having a portion configured to secure the nasogastric tube and a portion configured to be repositionably coupled to the base layer to allow the nasogastric tube to be repositioned as desired without disrupting the base layer adhesion to skin, or requiring any portion of the base layer to be removed. The base layer can remain in position on the skin until it becomes necessary to change it or until the nasogastric tube is removed from the patient.

The systems of the present disclosure can be provided together as a kit, e.g., on one release liner, which can enhance manufacturability, packaging, ease-of-use and standardization of application procedures or techniques.

As a result, the systems of the present disclosure provide a repositionable coupling layer that can be repositioned as desired on a base layer that remains stably adhered to the skin until the entire system is to be changed or removed. The repositionable coupling layer secures the nasogastric tube on the patient's nose in order to keep it well placed. This allows site evaluation and helps reduce skin damage, as well as nostril pressure ulcers.

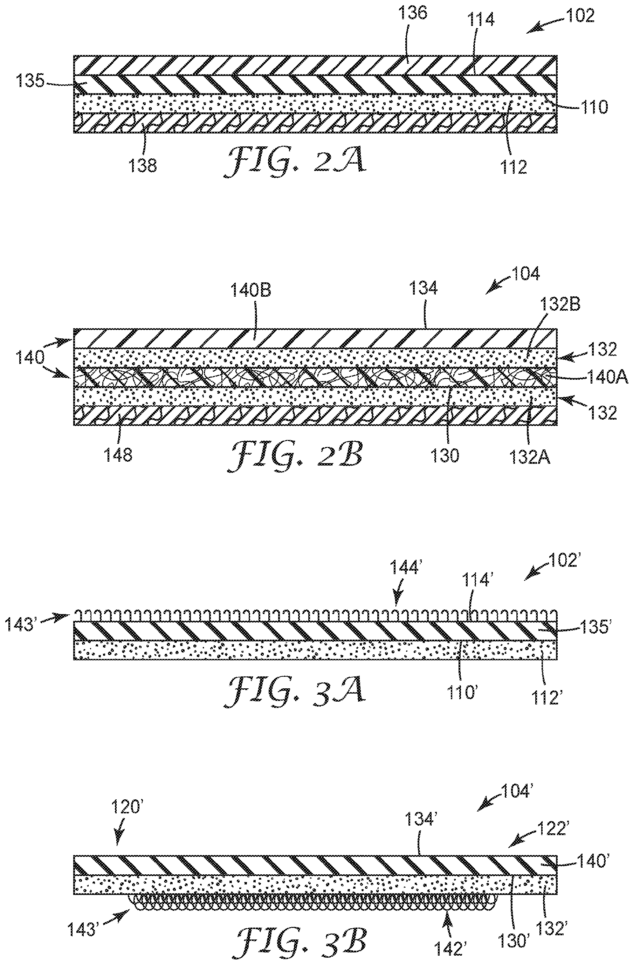

In some embodiments, the base layer can include a release agent (e.g., a release coating) on its back side to which an adhesive on the coupling layer can be adhered to ensure that the coupling layer (or at least a portion thereof) can be repositionable on the base layer. Alternatively or additionally, in some embodiments, the base layer can include a first mating surface of a mechanical fastener (e.g., hooks) on its back side to which a second mating surface of the mechanical fastener on the coupling layer (e.g., loops) can be repositionably engaged. This will be described in greater detail below with reference to FIGS. 3A and 3B.

### Definitions

The term "a", "an", and "the" are used interchangeably with "at least one" to mean one or more of the elements being described.

The term "and/or" means either or both. For example "A and/or B" means only A, only B, or both A and B.

The terms "including," "comprising," or "having," and variations thereof, are meant to encompass the items listed thereafter and equivalents thereof as well as additional items.

Unless specified or limited otherwise, the terms "connected" and "coupled," and variations thereof, are used broadly and encompass both direct and indirect connections and couplings.

The terms "layer," "sheet," and "dressing," or variations thereof, are used to describe an article having a thickness that is small relative to its length and width.

The terms "polymer" and "polymeric material" refer to both materials prepared from one monomer such as a homopolymer or to materials prepared from two or more monomers such as a copolymer, terpolymer, or the like. Likewise, the term "polymerize" refers to the process of making a polymeric material that can be a homopolymer, copolymer, terpolymer, or the like. The terms "copolymer" and "copolymeric material" refer to a polymeric material prepared from at least two monomers.

The term "repositionable" refers to the ability of an article or surface to be, at least initially, repeatedly coupled to (e.g., adhered to) and removed from a surface or substrate without substantial loss of coupling capability (e.g., adhesion) and without damage to either surface (e.g., article or underlying substrate) being coupled together. For example, a coupling layer of the present disclosure can be repositionable on a base layer of the present disclosure if the base layer and the coupling layer can be removed, or decoupled, from one another without causing damage to the base layer or the coupling layer. By way of example, some pressure-sensitive adhesives and mechanical fasteners are repositionable.

The phrase "mechanical fastener" or "touch fastener" generally refers to a fastener that includes two mating, or engagement, surfaces configured to be applied to one another, each mating surface having a plurality of engagement structures or features, such that engagement structures on one mating surface are configured to engage with the engagement structures on the opposing mating surface. In some embodiments, the mechanical fastener can include two flexible mating strips or layers. In some embodiments, the mechanical fastener can include a first mating surface comprising tiny, stiff protrusions shaped like hooks that are configured to engage a second mating surface comprising pliable loops (i.e., a "hook and loop fastener," or "hook and pile fastener"). In some embodiments, the mechanical fastener can include inter-engaging hooks (e.g., self-engaging hooks) on both mating surfaces (i.e., a "hook and hook fastener" or a "self-engaging hook fastener").

"Peel force" refers to the force needed to "peel" one surface from another surface at an angle with respect to the plane between the surfaces. Adhesive peel force can be measured using the ASTM method referenced in the "*Adhesives*" section below. Peel force between mating surfaces of a mechanical fastener can be measured using ASTM D5170-98 (2015) - Standard Test Method for Peel Strength ("T" Method) of Hook and Loop Touch Fasteners.

"Shear strength" (or "shear force") refers to the resistance to forces that cause, or tend to cause, two contiguous parts of a body to slide relatively to each other in a direction parallel to their plane of contact. That is, shear strength is the amount of force required to move one surface relative to another surface when the two surfaces are pulled in opposite directions parallel to their plane of contact. Adhesive shear force can be measured using the ASTM method referenced in the "*Adhesives*" section below. Shear force between mating surfaces of a mechanical fastener can be measured using ASTM D5169-98(2015) - Standard Test Method for Shear Strength (Dynamic Method) of Hook and Loop Touch Fasteners.

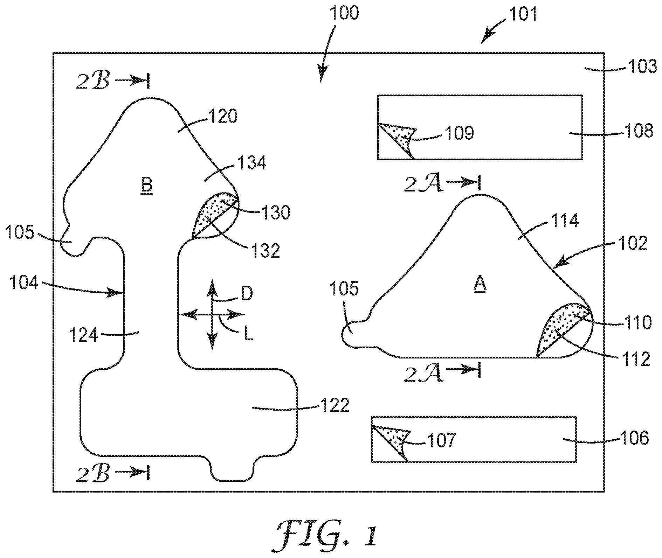

FIG. 1 illustrates a nasogastric tube securement system 100 according to one embodiment of the present disclosure. By way of example only, the system 100 is shown as a kit 101 comprising four elements of the system 100 all provided on one release liner 103. Additional details regarding release liners of the present disclosure are described in greater detail below under the section entitle, "*Release Liners*." As shown in FIG. 1, the nasogastric tube securement system can include a base layer, sheet or dressing 102, a coupling layer, sheet or dressing 104, and one or more additional auxiliary layers, sheets or tapes. The base layer 102 and the coupling layer 104 can be flexible sheets.

Specifically, by way of example only, the system 100 is shown as including a first tape strip 106 and a second tape strip 108. The first tape strip 106 can include securing means, e.g., a securing adhesive 107, configured to adhere to the nasogastric tube (e.g., an outer surface thereof) and configured to be wrapped about at least a portion of a circumference of the nasogastric tube to mark a desired depth of insertion into a subject's nostril.

The second tape strip 108 can include a skin-contact adhesive 109 and can be configured to be adhered to another portion of the subject's skin (e.g., on the face) to hold a portion of the length of the nasogastric tube out of the way to inhibit accidental tensions in the nasogastric tube or accidental removal of the nasogastric tube from the nostril or nasal cavity.

The first tape strip 106 and the second tape strip 108 are shown as being elongated and rectangular by way of example; however, it should be understood that the first tape strip 106 and the second tape strip 108 can have any shape suitable for marking the nasogastric tube and for adhering a portion of the tube to the subject's skin, respectively.

With continued reference to FIG. 1, the base layer 102 can be configured (e.g., dimensioned, shaped, formed of appropriate materials, etc.) to be adhered to the skin on the top of a nose (e.g., a human nose). The base layer 102 can include a first (e.g., bottom) major surface 110 comprising a skin-contact adhesive 112 and a second (e.g., top) major surface 114 opposite the first major surface.

Additional details regarding securing adhesives and skin-contact adhesives of the present disclosure are described in greater detail below under the section entitled, "*Adhesives*."

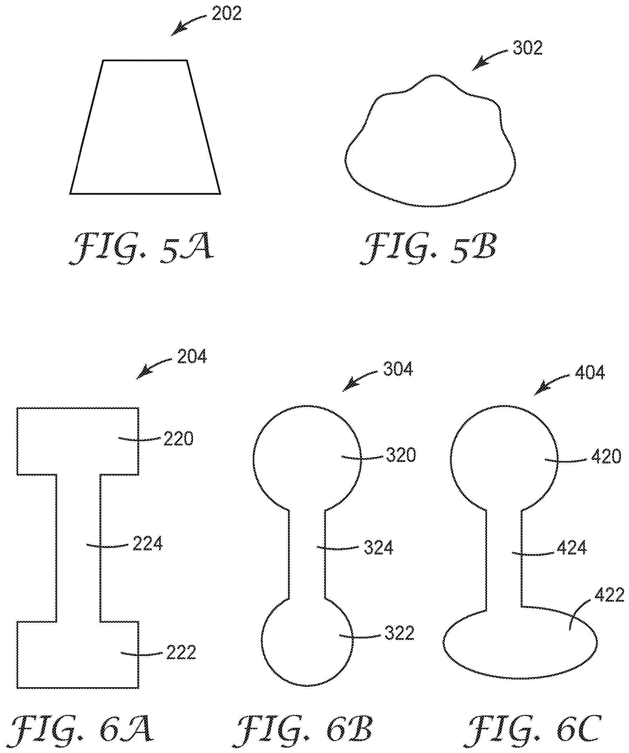

The base layer 102 can be shaped to be conducive to covering a nose, and particularly, a human nose. By way of example only, the base layer 102 of FIG. 1 has a generally triangular shape comprising a center portion that can overlap and adhere to the bridge of a nose, and two side flank portions extending out from the center portion configured to overlap and adhere to the sides of a nose, providing a secure and reliable base for the system 100. Other shapes are possible to achieve a stable and secure base, as described below with respect to FIGS. 5A and 5B. In addition, the base layer 102 may be provided in various sizes to accommodate different populations, e.g., smaller sizes for children.

The coupling layer 104 can include a first bulbous end (or "first end" or "nose securing portion" or "base layer coupling portion") 120; a second bulbous end (or "second end" or "nasogastric tube securing portion") 122; and a middle section (or "bridge" or "connector") 124. In some embodiments, the coupling layer 104 consists essentially of the first end 120, the second end 122, and the middle section 124. In some embodiments, the first end 120 and the second end 122 can be referred to as "bulbous" because of their protruding and expanded areas or shapes, relative to the narrower middle section 124.

The first end 120 can include coupling means configured to be repositionably coupled to the second major surface 114 of the base layer 102. The second end 122 can be configured to secure a nasogastric tube, i.e., can include securing means for reliably coupling the nasogastric tube. In some embodiments, it can be desirable for the second end 122 to not only securely hold the nasogastric tube for a desired duration of time but to also allow relatively easy removal of the nasogastric tube. As shown, the middle section 124 connects the first end 120 and the second end 122 and is elongated along or parallel to a longitudinal direction D extending between the first end 120 and the second end 122. As a result, the first end 120 is spaced a distance (i.e., a longitudinal distance) from the second end 122.

Furthermore, by way of example only, in some embodiments, the base layer 102 and/or the coupling layer 104 (e.g., the first end 120) can include one or more tabs 105, which can facilitate removal of the base layer 102 and/or the coupling layer 104 from the release liner 103. In addition, or alternatively, such tabs 105 can enhance removal of the base layer 102 from skin after use, and/or can enhance removal of the coupling layer 104 from the base layer 102 during use (e.g., for repositioning as necessary) or after use. To facilitate easy grasping of tabs 105, an additional section of release liner may be provided under only the portion 105.

In the embodiment illustrated in FIG. 1, the first end 120 and the second end 122 are each wider than the middle section 124 in a lateral, or transverse, direction L that is oriented substantially perpendicularly with respect to the longitudinal direction D. Said another way, in some embodiments, the first end 120 and the second end 122 each have a width (i.e., an ultimate lateral dimension) in the lateral direction L that is greater than that of the middle section 124. Particularly, in the embodiment of FIG. 1, the second end 122 of the coupling layer 104 is wider in the lateral direction L than the first end 120. In some embodiments, as shown in FIG. 1, the first end 120 and the second end 122 of the coupling layer 104 can each be at least two times wider than the middle section 124. The wider second end 122 can ensure a sufficient width for wrapping (or folding) about a circumference of a nasogastric tube to enhance the security of the nasogastric tube, without requiring that the entire coupling layer have this width, which could result in an overall large, cumbersome and bulky structure. Furthermore, the relative sizing between the second end 122, the first end 120 and the middle section 124 can ensure that the first end 120 can be sized appropriately for coupling to the nose (i.e., sized to be coupled over the nose via the base layer 102) and can ensure that the middle section 124 can be sufficiently narrow to bridge the first end 120 and the second end 122 without adding bulk, complexity or reducing patient comfort. In addition, the relative sizing can ensure that the middle section 124 is sufficiently long (i.e., in the longitudinal direction D) to provide adequate longitudinal extension between the first end 120 and the second end 122 to avoid pressure on the nostril.

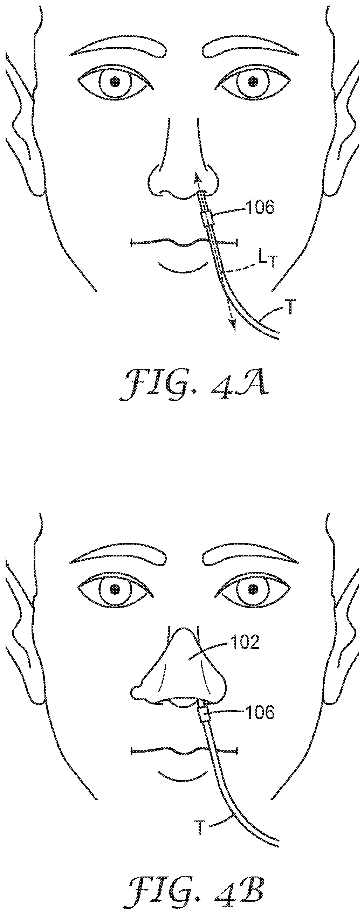

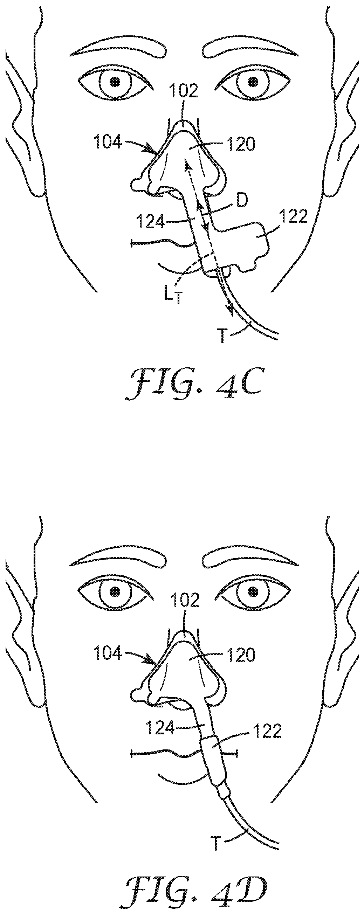

In some embodiments, the coupling layer 104 (e.g., the middle section 124) can be configured, such that, in use, the longitudinal direction D along which the middle section 124 is elongated is oriented substantially along or parallel to a longitudinal direction L_{T} (see FIG. 4A) of a nasogastric tube T to be secured by the system 100. In some embodiments, the coupling layer 104 (e.g., the middle section 124) can be configured, such that, in use, the longitudinal direction D is oriented substantially along or parallel to a bridge of the nose when the system is coupled to the nose. Furthermore, as shown in FIG. 4C, which is described in greater detail below, in some embodiments, the width of the first end 120 of the coupling layer 104 and/or the width of the base layer 102 can be oriented, in use, substantially laterally with respect to a bridge of a nose when the system 100 is coupled to the nose.

In some embodiments, the middle section 124 can be longer in the longitudinal direction D than either of the first end 120 or the second end 122 to ensure that the second end 122 secures the nasogastric tube at a longitudinal position that will not directly impinge on the nose, nostril, or otherwise, pull or cause tension on the first end 120 of the coupling layer 104, the base layer 102, the nose, or the nostril.

The coupling layer 104 is generally "L" shaped, or generally has an "L" configuration, i.e., generally takes the shape of a capital English letter "L." For example, in such embodiments, the middle section 124 can connect to the second end 122 at a location that is near one of its lateral ends. That is, in some embodiments, the middle section 124 and the second end 122 of the coupling layer 104 can generally form an "L" shape.

As shown in FIG. 1, the middle section is linear. Furthermore, in some embodiments, the middle section 124 can have a uniform width along its length between the first end 120 and the second end 122.

The coupling layer 104 can include a first major surface 130 configured to be positioned toward the patient (i.e., toward the patient's skin and nose) and toward a nasogastric tube to be secured by the system 100, and a second major surface 134 configured to face away from the patient (i.e., away from the patient's skin and nose) and the nasogastric tube, the second major surface 134 being opposite the first major surface 130. The coupling layer 104 can include one or more adhesives on the first major surface 130, such as the securing adhesive 132 shown in FIG. 1. For example, in some embodiments, the first major surface 130 can include a different adhesive in the region of the first end 120 than in the region of the second end 122, but this need not be the case. In some embodiments, the first major surface 130 can include first coupling means located at least partially in the first end 120 configured for repositionable coupling to the second major surface 114 of the base layer 102, and second coupling means located at least partially in the second end 122 configured to secure a nasogastric tube. However, in some embodiments, the first major surface 130 of the coupling layer 104 can include one coupling means (e.g., one securing adhesive). In such embodiments, the second major surface 114 of the base layer 102 can be modified to include a release agent (e.g., in the form of a release layer, a release coating, or a combination thereof) configured to release the securing adhesive on the first major surface 130 of the coupling layer 104, such that the first end 120 of the coupling layer 104 is repositionable on the base layer 102 as needed.

In some embodiments, as described below with respect to FIGS. 3A and 3B, a mechanical fastener can be employed (e.g., between the second major surface 114 of the base layer 102 and the first major surface 130 of the first end 120 of the coupling layer 104) in addition to the securing adhesive 132, or as an alternative thereto. For example, one mating surface of a mechanical fastener can be employed on the first major surface 130 of the coupling layer 104, and the complementary mating surface of the mechanical fastener can be employed on the second major surface 114 of the base layer 102 to achieve strong but repositionable coupling between the base layer 102 and the coupling layer 104.

However, even though the first end 120 of the coupling layer 104 (and particularly, the first major surface 130 thereof) and the base layer 102 (and particularly, the second major surface 114 thereof) are configured such that the first end 120 of the coupling layer 104 is repositionable on the base layer 102, the engagement (e.g., adhesion) between the first end 120 of the coupling layer 104 and the base layer 102 also still needs to be sufficiently strong in order to provide reliable securement of a nasogastric tube for the desired period of time.

That is, whether an adhesive, a mechanical fastener, or another coupling means is employed between the base layer 102 and the coupling layer 104 (i.e., the first end 120 thereof), the base layer 102 and the coupling layer 104, and particularly, the second major surface 114 of the base layer 102 and the first major surface 130 of the coupling layer 104, should be configured such that the peel force required to remove (i.e., peel) the coupling layer 104 from the base layer 102 is relatively low to allow easy repositioning as necessary, while the shear strength between the layers is relatively high to ensure adequate securement of the nasogastric tube. The present inventors discovered that by employing base layers and coupling layers specifically shaped and configured as described herein, they were able to achieve this balance of mechanical properties.

As shown in FIG. 1 by way of example only, in some embodiments, the base layer 102 can have a generally triangular shape, and particularly, generally has the shape of an equilateral triangle. The base layer 102 also has rounded corners for enhanced patient comfort. However, the base layer 102 can have any shape that is suitable for covering or wrapping over a nose, and particularly, the top of a nose. In some embodiments, the base layer 102 can have a shape that is suitable for covering a substantial portion of a nose, including the bridge of the nose and at least part of the lateral sides of the nose. Furthermore, in some embodiments, as shown in FIG. 1, the base layer 102 can have lateral symmetry, which can enhance the coupling to a nose. However, generally, the base layer 102 is also shaped so as not to extend downwardly over the tip or the nose or to otherwise interfere with a nasogastric tube to be secured by the system 100.

FIGS. 5A and 5B illustrate two examples of alternative shapes for base layers of the present disclosure. FIG. 5A shows a base layer 202 having a generally trapezoidal shape that is wider at its base (i.e., the portion to be positioned toward the tip of the nose) than it is at its top (i.e., the portion to be positioned away from the tip of the nose). FIG. 5B shows a base layer 302 having an irregular lobed shape that is wider at its base than at its top and that includes one or more scalloped rounded edges, which can enhance the conformability of the base layer 302 to a nose and/or patient comfort. While FIGS. 5A and 5B are illustrated to show other possible base layer shapes that can be employed, the three illustrated shapes (i.e., in FIGS. 1, 5A and 5B) are not exhaustive and other shapes that suitably cover the nose are also possible, including, but not limited to, oblong, circular, parallelogrammatic (e.g., square, rectangular), other suitable shapes, or combinations thereof. Any of the previous or following disclosure regarding base layers of the present disclosure refers to the base layer 102 of FIG. 1 for simplicity, but it should be understood that any such disclosure can also equally apply to the base layers 202 and 302 of FIGS. 5A and 5B.

As shown FIG. 1, in some embodiments, the first end 120 of the coupling layer 104 can have a shape that mimics the shape of the base layer 102, while also generally being smaller than the base layer 102, such that the area of the first end 120 can be contained within the area of the base layer 102 when the first end 120 is coupled to the base layer 102. Such a relationship between the shape and size of the first end 120 of the coupling layer 104 and the base layer 102 can enhance the coupling between the first end 120 and the base layer 102. The first end 120 of FIG. 1 has a generally triangular shape, and particularly, generally has the shape of an equilateral triangle. In addition, similar to the base layer 102, the first end 120 of the coupling layer 104 can also have rounded corners. However, similar to the base layer 102, the first end 120 of the coupling layer 104 can have any shape suitable for coupling to the base layer 102 and for also covering or wrapping over at least a portion of a nose, and particularly, the top of a nose. In some embodiments, the first end 120 of the coupling layer 104 can have a shape that is suitable for covering a substantial portion of a nose, including the bridge of the nose and at least part of the lateral sides of the nose. Furthermore, in some embodiments, as shown in FIG. 1, the first end 120 can have lateral symmetry, which can enhance the coupling to a nose. In addition, the coupling layer 104 can be configured to extend down from a bridge of the nose and over the front tip of the nose. Such a configuration can ensure that the nasogastric tube is secured in such a way that limits lateral pulling or tension on the nasogastric tube that could cause pressure ulcers on the nostril.

FIGS. 6A-6C illustrate three examples of alternative shapes for coupling layers of the present disclosure, although the embodiments of figure 6a, 6b are not part of the invention. FIG. 6A shows a coupling layer 204 having a generally rectangular first end 220 and second end 222 connected by a middle section 224, generally forming an "I" configuration. In addition, the coupling layer 204 has lateral symmetry, such that the middle section 224 connects to the first end 220 and the second end 222 at a lateral center thereof. Furthermore, each of the first end 220 and the second end 222 are shown by way of example as have the same width and length (i.e., in a longitudinal direction), such that the coupling layer 204 also has longitudinal symmetry about a longitudinal center.

FIG. 6B shows a coupling layer 304 having a generally circular first end 320 and second end 322 connected by a middle section 324, generally forming an "I" configuration. In addition, the coupling layer 304 has lateral symmetry, such that the middle section 324 connects to the first end 320 and the second end 322 at a lateral center thereof. By way of example only, the first end 320 is slightly larger (i.e., larger diameter circle) and has a larger area than the second end 322.

FIG. 6C shows a coupling layer 404 having a generally circular first end 420, an oblong second end 422 with its longer axis extending in a lateral direction, and a middle section 424 connecting the first end 420 and the second end 422. The second end 422 is wider (i.e., in a lateral direction) and shorter (i.e., in a longitudinal direction) than the first end 420, which can form a shape conducive to being wrapping about a circumference of a nasogastric tube. The middle section 424 connects to the first end 420 at a lateral center of the first end 420, but connects to the second end 422 at a lateral position located between a lateral center and a lateral end, such that the coupling layer 404 does not have lateral symmetry and such that the middle section 424 and the second end 422 generally form an "L" configuration.

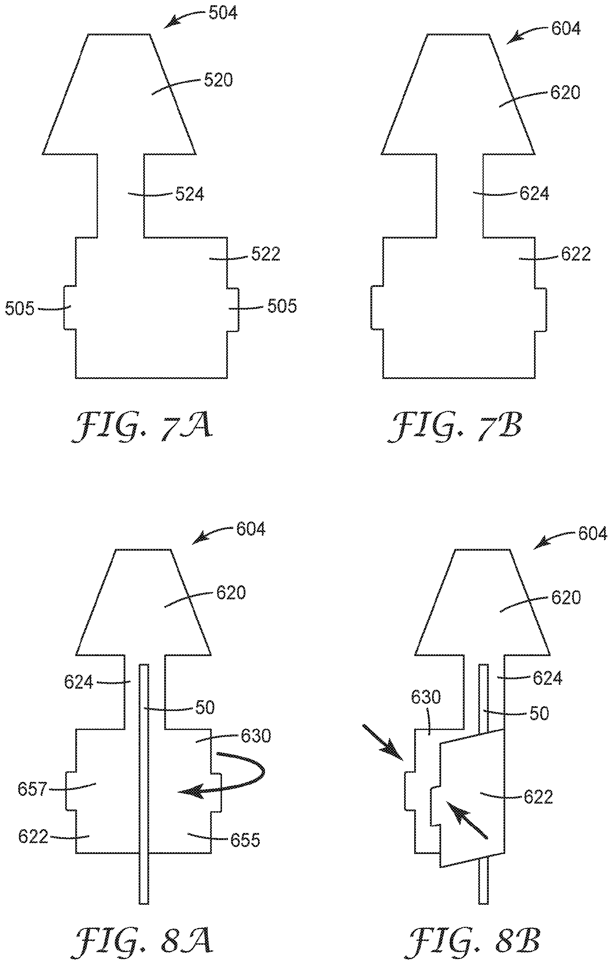

FIG. 7A illustrates a coupling layer 504 having a generally trapezoidal first end 520 that is wider at its base, a square second end 522 with side tabs 505, and a middle section 524 connecting the first end 520 and the second end 522. The side tabs 505 on the second end 522 can facilitate removing the coupling layer 504 from a release liner and/or can facilitate folding the second end 522 over a nasogastric tube. That is, the coupling layer 504, and particularly, the second end 522 can be configured to facilitate securing a nasogastric tube by merely folding the second end 522 laterally (e.g., in half), where the fold would be generally aligned with or parallel to a longitudinal direction along which the middle section 524 is elongated. The middle section 524 connects to the first end 520 at a lateral center of the first end 520, but connects to the second end 522 at a lateral position located between a lateral center and a lateral end, such that the coupling layer 504 does not have lateral symmetry and such that the middle section 524 and the second end 522 generally form an "L" configuration, or the mirror (reverse image) of the letter "L."

FIG. 7B illustrates a coupling layer 604 that is also configured for being folded over a nasogastric tube, similar to the coupling layer 504 of FIG. 7A. The coupling layer 604 includes a first end 620, a second end 622 and a middle section 624 that are substantially the same as the coupling layer 504 of FIG. 7A, except that the middle section 624 connects to the first end 620 and the second 622 at a lateral center thereof, such that the coupling layer 604 has lateral symmetry.

FIGS. 8A and 8B illustrate using the coupling layer 604 of FIG. 7B to secure a nasogastric tube 50. In the example, the exposed surface of the coupling layer 604 is a first major surface 630 that can include one or more coupling means, such as a securing adhesive, a mechanical fastener, or the like. Specifically, FIG. 8A shows how the nasogastric tube 50 can be aligned along a central longitudinal axis of the coupling layer 604, e.g., along at least a portion of the middle section 624 and the second end 622. The first end 620 can be coupled to a base layer on the nose before and/or after coupling the nasogastric tube 50. As further shown in FIG. 8A, the second end 622 can include a first lateral portion 655 and a second lateral portion 657 that each extend laterally with respect to the middle section 624. As a result, the first lateral portion 655 and the second lateral portion 657 can overlap one another when folded over the nasogastric tube 50.
FIG. 8B shows one example of how the second end 622 could be folded laterally over the nasogastric tube 50. Specifically, the first lateral portion 655 can be folded along a longitudinal line to overlap the nasogastric tube 50 and at least a portion of the second lateral portion 657. In addition, at least a portion of the middle section 624 could be folded laterally over the nasogastric tube 50, e.g., if sufficiently wide. In such embodiments, the first major surface 630 of the second end 622 can include a securing adhesive that can adhere to the nasogastric tube 50, as well as to itself over the nasogastric tube 50 when the first lateral portion 655 and the second lateral portion 657 are in an overlapping relationship. However, in some embodiments, the first lateral portion 655 of the first major surface 630 of the second end 622 can include a first mating surface of a mechanical fastener, and the second lateral portion 657 of the first major surface 630 can include the opposing second mating surface of the mechanical fastener. In some embodiments, a combination of adhesive, mechanical fastener, or other suitable coupling means can be employed.

While FIGS. 6A-7B are illustrated to show other possible coupling layer shapes that can be employed, the six illustrated shapes (i.e., in FIGS. 1, 6A-6C and 7A-7B) are not exhaustive and other shapes, or other combinations of first end and second end shapes, are also possible to provide suitable coupling between the first end of the coupling layer and the base layer while also providing sufficient distance between the first end and the second, as well as sufficient coupling between the second end and a nasogastric tube. Thus, various shapes can be employed as the first end, the second end, or both, including, but not limited to, trapezoidal, lobed, triangular, oblong, circular, parallelogrammatic (e.g., square, rectangular), other suitable shapes, or combinations thereof. Any of the previous or following disclosure regarding coupling layers of the present disclosure refers to the coupling layer 104 of FIG. 1 for simplicity, but it should be understood that any such disclosure can also equally apply to the coupling layers 204, 304 and 404 of FIGS. 6A-6C, as well as the coupling layers 504 and 604 of FIGS. 7A and 7B.

With continued reference to FIG. 1, in some embodiments, the base layer 102 can have a footprint area A, which is measured when the base layer 102 is in a flat configuration as shown in FIG. 1, e.g., before being applied to a nose. This area A is the overall footprint that the base layer 102 takes up on the release liner 103 and generally over a nose, when in use. The first end 120 of the coupling layer 104 can have a footprint area B. In some embodiments, the footprint area B of the first end 120 can be at least 0.3A (i.e., at least 30% of footprint area A); in some embodiments, at least 0.4A; in some embodiments, at least 0.5A; in some embodiments, at least 0.6A; in some embodiments, at least 0.7A; in some embodiments, at least 0.75A; in some embodiments, at least 0.8A; in some embodiments, at least 0.85A; in some embodiments, at least 0.9A; and in some embodiments, at least 0.95A. In some embodiments, the footprint area B of the first end 120 can be no greater than 0.98A; in some embodiments, no greater than 0.97A; in some embodiments, no greater than 0.95A. Increasing the footprint area B of the first end 120 of the coupling layer 104, relative to the footprint area A of the base layer 102, can enhance the coupling (e.g., shear strength) between the first end 120 and the base layer 102, which can enhance the securement of a nasogastric tube.

As shown in FIG. 2A which illustrates a side cross-sectional view of one embodiment of the base layer 102, in some embodiments, the base layer 102 can include a backing 135 that provides the first major surface 110 and the second major surface 114. In some embodiments, the base layer 102 can include a multi-layer structure, including a plurality of backings 135, and can optionally include additional adhesives located between adjacent backings 135. In such embodiments, the first major surface 110 of the base layer 102 can be provided by a lowermost backing, and the second major surface 114 of the base layer 102 can be provided by an uppermost backing in the multi-layer structure. That is, while only one backing 135 is shown in FIG. 2A, it should be understood that as many backings 135 (and, optionally, adhesives therebetween) can be employed in the base layer 102, as long as the exposed adhesive on the first major surface 110 of the overall base layer 102 is a skin-contact adhesive 112 suitable for being adhered to skin, and particularly to the skin on the nose.

Various additional details regarding backings of the present disclosure are described in greater detail below under the section entitled, "*Backings*."

In addition, in some embodiments, the multi-layer concept can also be used in the configuration of the kit 101 of FIG. 1, where, for example, the base layer 102 and the coupling layer 104 can be provided already overlapped on the release liner 103 (e.g., where a release agent on a top surface of one layer can serve as the release liner for another layer). In addition, or alternatively, in some embodiments, the kit 101 can optionally include an extra coupling layer 104 that can be supplied to guarantee an extra adjustment, if necessary, for a nasogastric tube. In some embodiments, the additional coupling layer 104, for example, can be supplied directly under the first coupling layer 104, thereby taking up no additional footprint area of the kit 101. Furthermore, in some embodiments, the first tape strip 106 and the second tape strip 108 can be provided in an overlapped configuration on the release liner 103. The cross-sectional multilayer configuration of such overlapped embodiments would be similar to the construction shown in FIG. 2B, which is described in greater detail below. Furthermore, in some embodiments, the kit 101 can include multiple coupling layers 104 of different shapes, types and/or sizes, such that the kit 101 provides several options for use, for example, depending on patient anatomy.

As shown in FIG. 2A, the base layer 102 can further include the skin-contact adhesive 112 on the first major surface 110, and a release agent (e.g., a release coating) 136 on the second major surface 114 of the backing 135. Such a release agent 136 can be selected to function as a release layer or liner for an adhesive (e.g., a securing adhesive) located on the first major surface 130 of the coupling layer 104, and particularly, for an adhesive located on the first major surface 130 in the first end 120 of the coupling layer 104. As further shown in FIG. 2A, in some embodiments, the base layer 102 can further include a release liner 138 (e.g., a paper liner comprising a release agent, e.g., silicone, for the skin-contact adhesive 112). However, in some embodiments, as shown in FIG. 1, the base layer 102 may be provided on the same release liner 103 as the rest of the system 100 and not include its own dedicated release liner 138.

In some embodiments, the release agent 136 can include a low adhesion (low adhesion backsize, or LAB) coating provided on the second major surface 114 of the base layer 102 at least in a region positioned to come into contact with the coupling layer 104. The low adhesion coating can allow the coupling layer 104 to be repositionable on the base layer 102 to the extent necessary. A description of a low adhesion backing material suitable for use with medical dressings of the present disclosure can be found in U.S. Patent Nos. 5,531,855 and 6,264,976.

In some embodiments, the backing 135 can be formed of a stretchable material (e.g., a stretchable nonwoven, woven, film, or combination thereof) that can provide gentle removal to minimize skin damage when the system 100 (and, particularly, the base layer 102 of the system 100) is removed. For example, in some embodiments, the base layer 102 can include a stretch release backing 135 (i.e., a backing 135 formed of a stretch release material) and skin-contact adhesive 112, such that while stretching, there is a distribution of tension force between the backing 135, the adhesive 112, and the skin, providing adhesive failures and reducing the tension applied on the skin as the base layer 102 is removed.

By way of example only, in some embodiments, the backing 135 and the skin-contact adhesive 112 can be provided by polyurethane stretchable nonwoven tape, such as the tape available as 3M™ CoTran™ 9699 Melt Blow Polyurethane Tape from 3M Company, St. Paul, MN, any of the materials AH of Table 1 in the Examples section below, other suitable tapes/backings, or a combination thereof.

In some embodiments, it can be advantageous for the base layer 102 to be formed of a relatively stretchy (e.g., elastic, viscoelastic, etc.) and conformable material, while the coupling layer 104 is formed of a relatively non-stretchy (e.g., inelastic, rigid, etc.) material. Such relative material properties can enhance patient comfort and/or facilitate removal of the base layer 102 from the skin, while also ensuring enough tensile strength in the coupling layer 104 to securely hold a nasogastric tube in a desired position without allowing the nasogastric tube to shift or cause undue pressure on the skin or nostril.

For example, in some embodiments, the base layer 102 can have a percent elongation at break (or maximum elongation) of at least 200%; in some embodiments, at least 250%; in some embodiments, at least 300%; in some embodiments, at least 400%; and in some embodiments, at least 500%.

In some embodiments, the coupling layer 104 can have a percent elongation at break of no greater than 100%; in some embodiments, no greater than 80%; in some embodiments, no greater than 75%; and in some embodiments, no greater than 50%.

Percent elongation at break can be measured using any standard tensile testing equipment known to those of ordinary skill in the art. One example of tensile testing is described in the Examples section.

As shown in FIG. 2B, in some embodiments, the coupling layer 104 can include a multi-layer structure (e.g., multi-layer tape or multiple tapes) comprising one or more backings 140 and one or more securing adhesives 132. As shown in FIG. 2B, the first major surface 130 of the coupling layer 104 can be provided by one backing 140, and the first major surface 130 can include a securing adhesive 132 configured to repositionably adhere to the release agent 136 on the second major surface 114 of the base layer 102, as well as adhere to a nasogastric tube (and, optionally, its own second major surface 134) to securely hold a nasogastric tube in place. By way of example only, in the embodiment illustrated in FIG. 2B, the first major surface 130 is provided by a first adhesive backing (e.g., tape) 140A, and the second major surface 134 is provided by a second adhesive backing (e.g., tape) 140B that is laminated over the first adhesive backing 140A. However, it should be understood that the illustrated laminate structure need not be employed and that the first major surface 130 and the second major surface 134 can be provided by one backing 140. While two backings 140 and securing adhesives 132 are shown in FIG. 2B by way of example, it should be understood that as few as one backing 140 and securing adhesive 132, or as many as structurally possible or necessary, can be employed.

That is, as shown in FIG. 2B, in some embodiments, the coupling layer 104 can be formed of the first backing 140A and a first securing adhesive 132A, and the second backing 140B and a second securing adhesive 132B that adheres the second backing 140B to the first backing 140A. By way of example only, in some embodiments, the first backing 140A and the first securing adhesive 132A can be provided by a polyethylene terephthalate (PET) nonwoven-acrylic adhesive tape, such as the tape available as 3M™ Tan Spunlaced Nonwoven Medical Tape 9916, 3M Company, St. Paul, MN In addition, in some embodiments, the second backing 140B and the second securing adhesive 132B can be provided by a polyethylene backing-acrylic adhesive tape, such as the tape available as 3M™ Blenderm™ Surgical Tape 1525, 3M Company, St. Paul, MN. Such a laminate structure can provide the necessary strength to the coupling layer 104 to secure a nasogastric tube and keep it in the correct position for the desired period of time. The first securing adhesive 132A functions as the exposed securing adhesive that will be adhered to the nasogastric tube. The specific tapes listed above are described by way of example; however, the coupling layer 104 can also include any of the materials I-M of Table 1 in the Examples section below, other suitable tapes/backings, or a combination thereof.

As further shown in FIG. 2B, the coupling layer 104 can further include a release liner 148 (e.g., a paper liner comprising release agent for the securing adhesive 132 exposed on the first major surface 130). However, in some embodiments, as shown in FIG. 1, the coupling layer 104 may be provided on the same release liner 103 as the rest of the system 100 and not include its own dedicated release liner 148.

Furthermore, in some embodiments, the second major surface 134 of the coupling layer 104 can include a release agent similar to the release agent 136 of the base layer 102 of FIG. 2A, described above. For example, the second major surface 134 can include a low adhesion (low adhesion backsize, or LAB) coating. Such a release agent on the second major surface 134 of the coupling layer 104 (e.g., at least in the region of the second end 122 of the coupling layer 104) can facilitate unwrapping the coupling layer 104 during the process of removing the system 100 and decoupling the system 100 from a nasogastric tube.

While only one securing adhesive 132 is shown as being present on the first major surface 130 of the coupling layer 104, it should be understood that in some embodiments, the first major surface 130 of the coupling layer 104 in at least a portion of the first end 120 can include a first securing adhesive (e.g., a less aggressive adhesive with a lower peel force on the second major surface 114 of the base layer 102), and the first major surface 130 in at least a portion of the second end 122 can include a second securing adhesive (e.g., a more aggressive adhesive with a higher peel force on the outer surface of the nasogastric tube) that is different from the first securing adhesive. In such embodiments, the middle section 124 can be free of adhesive, can include the first securing adhesive, can include the second securing adhesive, or can include a combination of the first securing adhesive and the second securing adhesive.

Furthermore, while the base layer 102 shown in FIG. 2A is shown as being the same size (i.e., length) as the coupling layer 104 of FIG. 2B, these two figures are not necessarily drawn to scale. Rather, as shown in FIG. 1, the coupling layer 104 would generally be longer than the base layer 102 (i.e., in the direction of the width of the page of FIGS. 2A and 2B), such that the first end 120 of the coupling layer 104 can be sized and positioned to overlap at least a portion of the base layer 102, while the second end 122 and at least a portion of the middle section 124 of the coupling layer 104 can extend beyond the area of the base layer 102 to access and secure a nasogastric tube, e.g., according to the relative sizes shown in FIG. 1.

FIGS. 3A and 3B illustrate a base layer 102' and a coupling layer 104', respectively, according to another embodiment of the present disclosure. For simplicity, no additional release liners are shown in FIGS. 3A and 3B. In addition, each of the base layer 102' and the coupling layer 104' are shown for simplicity as including only one backing - backings 135' and 140', respectively. However, it should be understood that one or both of the base layer 102 and the coupling layer 104 can be a multi-layer structure, as described above and as shown in FIG. 2B.

FIGS. 3A and 3B represent an example of repositionable coupling means between the base layer 102' and the coupling layer 104' that includes a mechanical fastener. As shown in FIG. 3A, the base layer 102' includes the backing 135' having first major surface 110' and second major surface 114', and a skin-contact adhesive 112' on the first major surface 110'. As shown in FIG. 3B, the coupling layer 104' includes the backing 140' having first major surface 130' and second major surface 134', and a securing adhesive 132' on the first major surface 130'. The coupling layer 104' further includes a first mating surface 142' of a mechanical fastener 143' on the first major surface 130', which can be coupled (e.g., laminated) to the first major surface 130' via the securing adhesive 132'. By way of example only, the first mating surface 142' of the coupling layer 104' is shown as being formed of loops or pile, however, other mechanical fastener features can be used.

As further shown in FIG. 3A, the base layer 102' further includes a second mating surface 144' of the mechanical fastener 143' on the second major surface 114' that is configured to reversibly engage the first mating surface 142' of the coupling layer 104'. By way of example only, the second mating surface 144' is shown as being formed of hooks, however, other mechanical fastener features can be used. In some embodiments, the second mating surface 144' of the mechanical fastener 143' can be provided on the base layer 102' by laminating.

By way of example only, in some embodiments the backing 135' and skin-contact adhesive 112' can be provided by a polyethylene terephthalate (PET)-acrylic adhesive tape, available under the trade designation 3M™ Spunlaced Polyester Nonwoven Medical Tape 1776 from 3M Company, St. Paul, MN Other examples useful for providing the backing 135' and the skin-contact adhesive 112', include, but are not limited to, a polyethylene terephthalate (PET) nonwoven-acrylic adhesive tape, such as the tape available as 3M™ Tan Spunlaced Nonwoven Medical Tape 9916 from 3M Company, St. Paul, MN.; a rayon nonwoven tape, such as the tape available as 3M™ Microporous Tan Rayon Nonwoven Medical Tape 1533 from 3M Company, St. Paul, MN.; a suitable elastic backing with a gentle adhesive; or a combination thereof.

While the second mating surface 144' is shown as being coextensive with the second major surface 114' of the base layer 102', this need not be the case. Rather, in some embodiments, the second mating surface 144' can have an area less than a total surface area of the second major surface 114', e.g., such that the base layer 102' includes a border around all edges of the second major surface 114' that is free of the second mating surface 144'. Such embodiments can inhibit the potentially harder and more rigid mechanical fastener 143' component from irritating the skin on the nose, by providing a buffer all around where the backing 135' is free of the second mating surface 144'.

As shown in FIG. 3B, in some embodiments, the first mating surface 142' may not be coextensive with the first major surface 130' of the coupling layer 104'. For example, in some embodiments, the securing adhesive 132' may be exposed in a portion of the first end 120' (e.g., in some embodiments, in an area accounting for less than 10% of the total area of the first major surface 130' of the first end 120', in some embodiments, less than 20%, or in some embodiments, less than 30%). This can reduce the risk of the coupling layer 104' being inadvertently removed from the base layer 102' during use, enhancing the coupling between the first end 120' of the coupling layer 104' and the base layer 102', while still allowing for repositioning of the coupling layer 104' on the base layer 102' as needed. In some embodiments, however, the entire first end 120' of the coupling layer 104' can include the first mating surface 142' of the mechanical fastener 143' (e.g., if the mechanical fastener 143' has a sufficiently aggressive engagement between the first mating surface 142' and the second mating surface 144').

Furthermore, as shown in FIG. 3B, at least a portion of the second end 122' can be free of the mechanical fastener 143', so that the securing adhesive 132' can be exposed for securing a nasogastric tube. In some embodiments, at least 80% of the second end 122' is free of the mechanical fastener 143', in some embodiments, at least 90%, and in some embodiments, at least 95%.

### Methods of using systems of the present disclosure to secure a nasogastric tube

FIGS. 4A-4J illustrate a method of securing a nasogastric tube using the nasogastric tube securement system 100 of FIG. 1. Before inserting a nasogastric tube T into a subject's nose, the length of the tube to be inserted to reach a desired depth can be measured. Then, the first tape strip 106 can be wrapped about a nasogastric tube T (e.g., about a circumference thereof) to mark the measured length, e.g., by adhering the securing adhesive 107 to the outer surface of the nasogastric tube T and continuing to wrap the first tape strip 106 over itself. Then, as shown in FIG. 4A, the nasogastric tube T can be inserted into a nostril to the desired depth (see FIG. 4B).

As shown in FIG. 4B, the base layer 102 can be applied to the subject's nose, i.e., to cover a substantial portion of the top surface of the nose. Particularly, the skin-contact adhesive 112 on the first major surface 110 of the base layer 102 can be adhered to the skin on the top of the nose.

As shown in FIG. 4C, the first end 120 of the coupling layer 104 can then be applied to the base layer 102. As shown in FIG. 4C, the securing adhesive 132 can be used to adhere the first major surface 130 of the first end 120 of the coupling layer 104 to the second major surface 114 of the base layer 102. Alternatively or additionally, as shown in FIGS. 3A and 3B, the first mating surface 142' of the mechanical fastener 143' on the first major surface 130' of the coupling layer 104' can be engaged with the second mating surface 144' on the second major surface 114' of the base layer 102'. The first end 120 of the coupling layer 104 can be positioned on the base layer 102 in such a way that the first end 120 is positioned within the area of the base layer 102 and is generally aligned with the base layer 102.

As shown in FIGS. 4C and 4D, the middle section 124 (e.g., a central longitudinal axis thereof) can be aligned with the nasogastric tube T such that the longitudinal direction D (or a central longitudinal axis) of the system 100 is generally aligned with the longitudinal direction L_{T} of the nasogastric tube T, and the second end 122 of the coupling layer 104 can be coupled to the nasogastric tube T. Specifically, based at least in part on the L-shaped configuration of the coupling layer 104, the shorter lateral portion of the second end 122 can be wrapped at least partially around the nasogastric tube T and then the longer later portion of the second end 122 can be wrapped around the nasogastric tube and the rest of the second end 122 to secure the nasogastric tube T in its desired position (see FIG. 4D). As also shown in FIG. 4D, in some embodiments, at least a portion of the middle section 124 can also be wrapped around (and optionally adhered to) the nasogastric tube T.

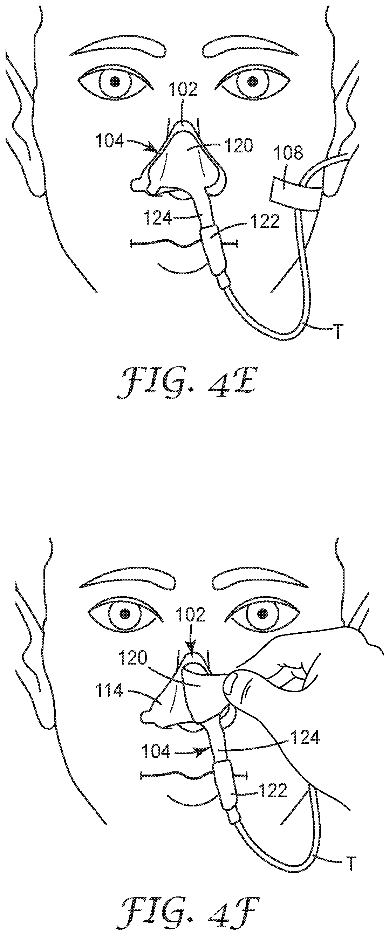

As shown in FIG. 4E, the second tape strip 108 can be used to adhere excess length of the nasogastric tube T to the patient's skin (e.g., face) in such a way that keeps the nasogastric tube T out of the way and inhibits unnecessary tensions or pulling forces on the nasogastric tube T. The remainder of the nasogastric tube T can then be threaded behind the patient's ear.

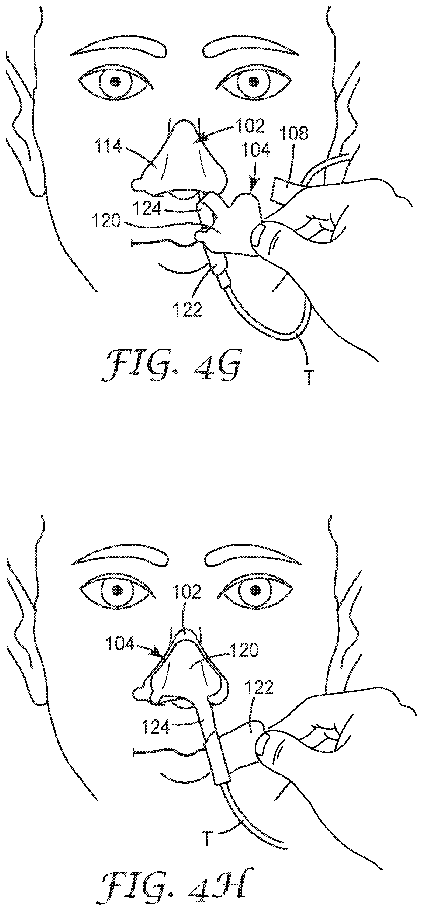

The system 100 as shown in FIG. 4E is therefore fully secured and can remain as shown until repositioning of the coupling layer 104 is necessary or until the system 100 needs to be removed or changed. FIGS. 4F and 4G illustrate how the first end 120 can be repositionable on the second major surface 114 of the base layer 102 while the rest of the coupling layer 104 remains in place on the nasogastric tube T and undisturbed. The first end 120 can then be readjusted as necessary (e.g., to remove any pressures on a nostril) and replaced back on the base layer 102.

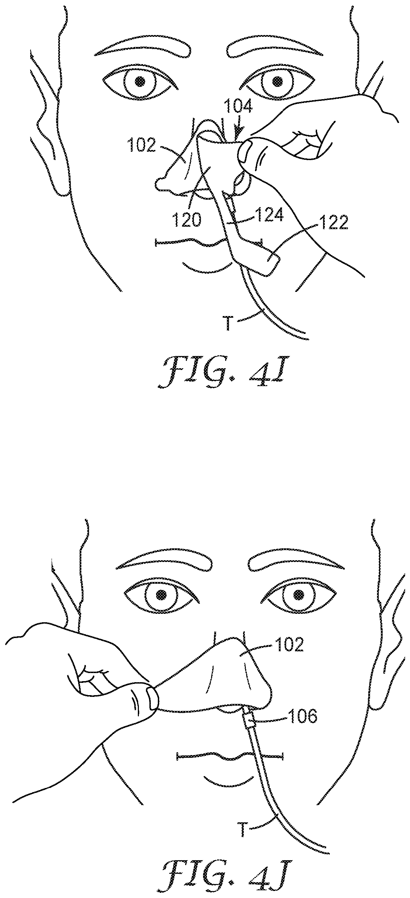

FIGS. 4H-4J illustrate how the system 100 can be removed, e.g., when it is desired to remove the nasogastric tube T. First, if employed, the second tape strip 108 can be peeled from the patient's skin. Then, as shown in FIG. 4H, a portion of the second end 122 can be grasped, and the second end 122 can be unwrapped from around the nasogastric tube T. As shown in FIG. 4I, the first end 120 of the coupling layer 104 can be lifted off of the base layer 102, and the whole coupling layer 104 can be removed (and disposed). While removing the second end 122 first can provide a less cumbersome removal method for removing the coupling layer 104, the first end 120 can be removed first instead. As shown in FIG. 4J, then only the base layer 102 remains on the nose. Then, in embodiments employing a stretch release material in the base layer 102, a corner or edge of the base layer 102 can be grasped, as shown in FIG. 4J, and pulled in order to gently remove the base layer 102 from the nose, reducing the risk of skin damage and increasing patient comfort. In embodiments not employing stretch release material in the base layer 102, the base layer 102 can be simply peeled from the nose.

### Backings

Suitable backings for base layers and coupling layers of the present disclosure can include, but are not limited to, one or more of a fabric, a woven fibrous web, a nonwoven fibrous web, a knit, a polymeric film, other familiar dressing materials, or combinations thereof. In some embodiments, the backing materials can include polymeric elastic films (e.g., transparent or non-transparent), and can include, but are not limited to, films formed of elastomeric polyurethanes, co-polyesters, polyethylenes, or combinations thereof. The backing can be a high moisture vapor permeable film, i.e., a backing with a relatively high moisture vapor transmission rate (MVTR). U.S. Patent No. 3,645,835 describes methods of making such films and methods for testing their permeability. The backing can be constituted of natural or synthetic sources of raw materials.

The backings of the present disclosure advantageously should transmit moisture vapor at a rate equal to or greater than human skin. In some embodiments, the backing can be adhesive-coated. In such embodiments, the adhesive-coated backing can transmit moisture vapor at a rate of at least 300 g/m²/24 hrs/37°C/100-10% RH, and in some embodiments, at least 700 g/m²/24 hrs/37°C/100-10% RH. The backing is generally conformable to anatomical surfaces. As such, when the backing is applied to an anatomical surface, such as a nose, it conforms to the surface even when the surface is moved.

The backing can be a flexible material. For example, the backing can be a film, paper, woven, knit, foam, nonwoven material, or a combination thereof, or one or more layers of film, paper, woven, knit, foam, nonwoven, or a combination thereof. In some embodiments, it can be desirable that at least a portion of the backing is formed of a transparent material to allow for viewing of underlying skin, a medical device, and/or a target site.

By way of example only, in some embodiments, the backing of a base layer of the present disclosure can be formed of a film available under the trade designation TEGADERM® from 3M Company, St. Paul, MN

### Release Liners

Release liners suitable for use with the systems of the present disclosure can include, but are not limited to, kraft papers, polyethylene, embossed polyethylene, polypropylene, polyester, or combinations thereof. Such liners can be coated with release agents, such as fluorochemicals, silicones, or other suitable low surface energy materials. Other adhesives and release liner combinations known to those of ordinary skill in the art can also be employed in the systems of the present disclosure. Examples of commercially available silicone coated release papers are POLYSLIK™, silicone release papers available from Rexam Release (Bedford Park, Ill.) and silicone release papers supplied by LOPAREX (Willowbrook, Ill.). Other non-limiting examples of such release liners commercially available include siliconized polyethylene terephthalate films, commercially available from H. P. Smith Co., and fluoropolymer coated polyester films, commercially available from 3M Company (St. Paul) under the brand "SCOTCHPAK™" release liners.

### Adhesives

As described above, the securing adhesives of the present disclosure (e.g., the securing adhesive 132 or 107 of FIG. 1 configured to be adhered a nasogastric tube) can have an adhesion that is higher than the skin-contact adhesives of the present disclosure (e.g., the skin-contact adhesive 112 or 109 of FIG. 1). In some embodiments, the securing adhesive and the skin-contact adhesive may be of the same or similar classes of adhesive, but have different adhesion levels. For example, the securing adhesive and/or the skin-contact adhesive may be an acrylate, silicone, urethane, hydrogel, hydrocolloid, natural rubber, or synthetic rubber. Adhesion can also be tuned through changes in adhesive composition, adhesive thickness, or adhesive surface area (e.g., by employing a pattern-coated adhesive).

"Adhesion" refers to the force required to separate an adhesive from an underlying substrate. Adhesion can be measured in a number of ways. For example, adhesion can be defined by peel force or shear force. In some embodiments, adhesion can be defined by peel adhesion using ASTM D3330/D3330M-04(2010). In some embodiments, adhesion can be defined by shear adhesion using ASTM D3654M-06(2011). Adhesion is dependent on the specific substrate being adhered to, as well as the time the pressure-sensitive adhesive (PSA) is allowed to dwell on the substrate.

For example, typical peel adhesion values exhibited by pressure-sensitive adhesives in medical dressings maybe in the range of 20 to 300 g/cm as measured from stainless steel. In some embodiments, at least 10% higher peel adhesion, as measured by ASTM D3330 / D3330M - 04(2010), of the securing adhesive over the skin-contact adhesive may realize the benefit of both securing to a nasogastric tube, while providing gentle adhesion to the skin.

In some embodiments, the securing adhesive can be an acrylate adhesive and the skin-contact adhesive can be a silicone adhesive. The term "acrylate" or "acrylate-based" or "acrylate-containing" refers to monomeric acrylic or methacrylic esters of alcohols. Acrylate and methacrylate monomers are referred to collectively herein as "acrylate" monomers. Materials that are described as "acrylate-based" or "acrylate-containing" contain at least some acrylate monomers and may contain additional comonomers.

Acrylate adhesives are well suited for securing adhesive dressings to medical articles (e.g., nasogastric tubes), or skin. The adhesion can be manipulated to have high adhesion or low adhesion. Generally, the adhesion between acrylate adhesives and another material will increase over time. This property makes acrylate adhesives well suited as the securing adhesive which is intended to secure a nasogastric tube.

Suitable acrylate adhesives that can be applied to skin such as the acrylate copolymers are described in U.S. Patent No. RE 24,906. In particular, a 97:3 iso-octyl acrylate:acrylamide copolymer. Another acrylate adhesive is a 70:15:15 isooctyl acrylate: ethyleneoxide acrylate:acrylic acid terpolymer, as described in U.S. Pat. No. 4,737,410 (Example 31). Other useful acrylate adhesives are described in U.S. Pat. Nos. 3,389,827, 4,112,213, 4,310,509, and 4,323,557.

The term "silicone" or "silicone-based" or "silicone-containing" refers to polymers that contain units with dialkyl or diaryl siloxane (-SiR₂O-) repeating units. The silicone-based polymers may be segmented copolymers or polysiloxanes polymers. The terms silicone and siloxane are used interchangeably.

Generally, silicone adhesives are able to effectively secure dressings and tape to skin and upon removal from the skin produce little or no skin damage. Typically, silicone adhesives do not adhere well to polymer-based substrates, like tubing or hardgoods, for example that are often present in nasogastric tubes. Thus, lack of strong adhesion to medical devices/tubing combined with the gentle removal of silicone adhesives from skin make these adhesives well suited as the skin-contact adhesive of the present disclosure.

Examples of suitable silicone adhesive systems can include, but are not limited to, products available under the following trade designations: Dow Corning MG 7-9850, Wacker SILPURAN® 2110 and 2130, Bluestar SILBIONE® RT Gel 4317 and 4320, Nusil MED-6345 and 6350. Other examples of suitable silicone adhesives are disclosed in PCT Publications WO2010/056541, WO2010/056543 and WO2010/056544.

For skin-contact adhesives, it is desirable that the adhesive is able to transmit moisture vapor at a rate greater to or equal to that of human skin. While such a characteristic can be achieved through the selection of an appropriate adhesive, it is also contemplated that other methods of achieving a high relative rate of moisture vapor transmission may be used, such as perforating the adhesive or pattern coating the adhesive, as described in U.S. Pat. No. 4,595,001 and U.S. Pat. App. Pub. 2008-0233348 (now U.S. Pat. No. 7,947,366) . Each of the securing or skin-contact adhesive can optionally be applied in a discontinuous manner.

Each embodiment shown in the figures is illustrated as a separate embodiment for clarity in illustrating a variety of features of the nasogastric tube securement systems of the present disclosure. However, it should be understood that any combination of elements and features of any of the embodiments illustrated in the figures and described herein can be employed in the nasogastric tube securement systems of the present disclosure.

In addition, various other features and elements can be employed in the nasogastric tube securement systems of the present disclosure, such as those disclosed in co-pending U.S. Application Nos. 62/208058 (Attorney Docket No. 76855US002); 62/208060 (Attorney Docket No. 76856US002); 62/208065 (Attorney Docket No. 76857US002); and 62/208069 (Attorney Docket No. 7685 8US002).

It is to be understood that the invention is not limited in its application to the details of construction and the arrangement of components set forth in the above description or illustrated in the accompanying drawings. Also, it is to be understood that the phraseology and terminology used herein is for the purpose of description and should not be regarded as limiting. It is to be further understood that other embodiments may be utilized, and structural or logical changes may be made without departing from the scope of the present disclosure.

The embodiments described above and illustrated in the figures are presented by way of example only and are not intended as a limitation upon the concepts and principles of the present disclosure. As such, it will be appreciated by one having ordinary skill in the art that various changes in the elements and their configuration and arrangement are possible without departing from the scope of the present disclosure.

The following working examples are intended to be illustrative of the present disclosure and not limiting.

### EXAMPLES

### Materials

Materials utilized in the Examples are shown in Table 1.

**Table 1. Materials List**

| **Material** | **Description** | **Source** |
|---|---|---|
| A - Foam Tape | 3M™ Polyethylene Foam Medical Tape 1774W, 510 micron, closed cell, polyethylene foam backing, coated with 60 micron thick pressure sensitive acrylate adhesive. | 3M Company, St. Paul, MN |
| B - PU-NW Tape | 3M™ CoTran™ 9699 Melt Blown Polyurethane Tape: 254 micron thick polyurethane/polyethylene backing coated with a gentle medical acrylate adhesive | 3M Company, St. Paul, MN |
| C - PU Film Tape | 3M™ Polyurethane Tape 9834; 20 ,micron polyurethane film with 25 micron thick gentle medical acrylate adhesive | 3M Company, St. Paul, MN |
| D - coPET Film Drape | 3M™ Steri-Drape™ 2 incise drape: 25 micron thick elastomeric copolyester backing coated with 51 micron thick pressure sensitive acrylate adhesive. | 3M Company, St. Paul, MN |
| E - Si Film | SILPURAN™ Film 2030; medical grade silicone film, 100 micron thick | Wacker Chemie AG, Munich Germany |
| F- PE Tape | 3M™ Blenderm™ Surgical Tape 1525 - Polyethylene backing coated with gentle medical acrylate adhesive | 3M Company, St. Paul, MN |
| G ― LDPE Film Drape | 3M™ Steri-Drape™ incise drape: 30 micron thick low density polyethylene backing coated with 51 micron thick pressure sensitive acrylate adhesive | 3M Company, St. Paul, MN |
| H ― coPET-AM Drape | 3M™ Ioban™ 2 Antimicrobial Incise Drape; 25 micron thick elastomeric copolyester backing coated with 51 micron thick iodophor impregnated (antimicrobial) pressure sensitive acrylate adhesive | 3M Company, St. Paul, MN |
| I ― PET-NW Tape | 3M™ Spunlaced Polyester Nonwoven Medical Tape 1776: polyester backing, coated with a medical, pressure sensitive acrylate adhesive | 3M Company, St. Paul, MN |
| J- RA-NW Tape | 3M™ Rayon Acetate Woven Medical Tape 1538; Rayon acetate woven cloth backing coated with a medical, pressure sensitive acrylate adhesive | 3M Company, St. Paul, MN |
| K- RA-MP NW Tape | 3M™ MICROPORE 1530 Surgical Tape; microporous rayon nonwoven backing coated with a medical, pressure sensitive acrylate adhesive | 3M Company, St. Paul, MN |
| L ― CAT Tape | 3M™ Cloth Adhesive Tape 2950; high strength cotton backing coated with a medical, pressure sensitive acrylate adhesive | 3M Company, St. Paul, MN |
| M-NW fiber strip | 3M™ Steri-Strip Skin Closure 1548; nonwoven backing, fiber reinforced with a medical, pressure sensitive acrylate adhesive | 3M Company, St. Paul, MN |

### Test Methods

### Tensile Test Method

Percent elongation was measured using a Universal test machine available from Kratos Industrial Equipment Ltda., BR, model K2000MP with a load cell of 20 kgf (196 N), depending on the properties of the backing to be tested, and with the gauge distance and the kart speed set according to the backing characteristics, as set forth in Table 2 below.

**Table 2. Gauge Distance and Test Speed for Elongation Testing**

| **Conditions** | **Distance between gauges** | **Test speed** |
|---|---|---|
| <100% Elongation | 100 mm | 100 mm/min |
| between 100 -400% | 50 mm | 200 mm/min |
| >400% | 20mm | 200 mm/min |

### Results

### Tensile Strength & Percent Elongation

Various backings or tapes useful for base layers and coupling layers of the present disclosure were tested according to the Tensile Test Method to determine the Tensile Strength (kgf; N) and Percent Elongation at break (%). Examples 1-8 represent relatively elastic backings having a percent elongation of at least 100% that can be used as base layers of the present disclosure. Examples 9-13 represent relatively non-elastic backings having a percent elongation of less than 100% that can be used as coupling layers of the present disclosure. Results are shown in Tables 3 and 4.

**Table 3. Tensile Strength & Percent Elongation for Base Layer backings**

| **Example** | **Base Layer Backing** | **Tensile Strength at break kgf (N)** | **Elongation max. (%)** |
|---|---|---|---|
| **1** | A - Foam Tape (n=10) | 2.36 (23.1 N) | 491.14 |
| **2** | B - PU-NW Tape (n=10) | 2.35 (23.0 N) | 485.54 |
| **3** | C - PU Film Tape (n=10) | 3.31 (32.5 N) | 586.49 |
| **4** | D - coPET Film Drape (n=10) | 2.62 (25.7 N) | 717.65 |
| **5** | E - Si Film (n=6) | 0.98 (9.6 N) | 472.48 |
| **6** | F - PE Tape (n=10) | 4.21 (41.3 N) | 204.32 |
| **7** | G - LDPE Film Drape (n=10) | 1.86 (18.2 N) | 229.46 |
| **8** | H - coPET-AM Drape (n=10) | 3.07 (30.1 N) | 835.55 |

**Table 4. Tensile Strength & Percent Elongation for Coupling Layer backings**

| **Example** | **Coupling Layer Backing** | **Tensile Strength at break kgf (N)** | **Elongation max. %** |
|---|---|---|---|
| **9** | I- PET-NW Tape (n=10) | 8.29 (81.3 N) | 41.58 |
| **10** | J - RA-NW Tape (n=10) | 16.54 (162.2 N) | 21.20 |
| **11** | K - RA-MP NW Tape (n=10) | 3.58 (35.1 N) | 13.02 |
| **12** | L - CAT Tape (n=9) | 13.65 (133.9 N) | 5.90 |
| **13** | M - NW fiber strip (n=10) | 10.33 (101.3 N) | 35.40 |

Various features and aspects of the present disclosure are set forth in the following claims.

## Claims

1. A nasogastric tube securement system (100), the system comprising:
a base layer (102, 102') configured to be adhered to a nose, the base layer (102, 102') having a first major surface (110, 110') comprising a skin-contact adhesive (112, 112') and a second major surface (114, 114') opposite the first major surface (110, 110'), the base layer (102, 102') having a footprint area of A; and
a coupling layer (104, 104', 404, 504, 604) comprising
a first end (120, 120', 420, 520, 620) comprising coupling means configured to be repositionably coupled to the second major surface (114, 114') of the base layer (102, 102'), the first end (120, 120') having a footprint area of at least 0.3A, wherein a width of the first end (120, 120', 420, 520, 620) of the coupling layer (104, 104', 505, 604) has a shape suitable for covering a bridge of the nose and at least a part of the lateral sides of the nose,
a second end (122, 122', 422, 522, 622) configured to secure a nasogastric tube (T), and
a linear middle section (124, 424, 524, 624) connecting the first end (120, 120', 420, 520, 620) and the second end (122, 122', 422, 522, 622), the middle section (124, 424, 524, 624) elongated along a longitudinal direction (D), and;
wherein the first end (120, 120', 420, 520, 620) and the second end (122, 122', 422, 522, 622) are each wider than the middle section (124, 424, 524, 624) in the lateral direction (L) that is oriented substantially perpendicularly with respect to the longitudinal direction (D), wherein at least the middle section (124, 424, 524, 624) and the second end (122, 122', 422, 522, 622) of the coupling layer (104, 104', 404, 504, 604) generally form an "L" shape, and wherein the coupling layer (104, 104', 404, 504, 604) is asymmetrical about the longitudinal direction (D).

2. The system of claim 1, wherein the second end (122, 122', 422, 522, 622) of the coupling layer (104, 104', 404, 504, 604) is wider in the lateral direction (L) than the first end (120, 120', 420, 520, 620).

3. The system of claim 1 or 2, wherein the coupling layer (104, 104', 604) includes
a first major surface (130, 130', 630) configured to be coupled to the second major surface (114, 114') of the base layer (102, 102'), the first major surface (130, 130', 630) comprising an adhesive (132, 132'), and
a second major surface (134, 134') opposite the first major surface (130, 130', 630), wherein the second major surface (134, 134') of at least a portion of the coupling layer (104, 104', 604) includes a release agent for the adhesive (132, 132').

4. The system of any of claims 1-3, wherein the coupling layer (104, 104', 604) includes a first major surface (130, 130', 630), wherein the coupling layer (104, 104', 604) further includes an adhesive (132, 132') on the first major surface (130, 130', 630), and wherein the second major surface (114, 114') of the base layer (102, 102') includes a release agent (136) for the adhesive (132, 132') on the first major surface (130, 130', 630) of the coupling layer (104, 104'. 604).

5. The system of any of claims 1-4, wherein the coupling layer (104') includes a first major surface (130'), wherein the coupling layer (104') includes a first mating surface (142') of a mechanical fastener (143') on the first major surface (130'), and wherein the second major surface (114') of the base layer (102') includes a second mating surface (144') of the mechanical fastener (143') configured to engage the first mating surface (143').

6. The system of any of claims 1-5, wherein the first end (120, 120', 420, 520, 620) and the second end (122, 122', 422, 522, 622) of the coupling layer (104, 104', 404, 504, 604) are each laterally centered with respect to the middle section (124, 424, 524, 624) of the coupling layer (104, 104', 404, 504, 604).

7. The system of any of claims 1-6, wherein the middle section (124, 424, 524, 624) of the coupling layer (122, 122', 422, 522, 622) has a uniform width.

8. The system of any of claims 1-7, wherein the first end (120, 120', 420, 520, 620) of the coupling layer (104, 104', 404, 504, 604) has a footprint area of at least 0.5A.

9. The system of any of claims 1-8, wherein the first end (120, 120', 420, 520, 620) of the coupling layer (104, 104', 404, 504, 604) has a shape that mimics the shape of the base layer (102, 102').

10. The system of any of claims 1-9, wherein the second end (122, 122', 422, 522, 622) of the coupling layer (104, 104', 404, 504, 604) includes a first major surface (130, 130', 630) comprising a securing adhesive (132, 132'), such that the securing adhesive (132, 132') on the first lateral portion (655) is positioned to adhere to the securing adhesive (132, 132') on the second lateral portion (657) when the first lateral portion (655) and the second lateral portion (657) are in an overlapping relationship.

11. The system of any of claims 1-10, wherein the base layer (102, 102') has a percent elongation of at least 200%, and wherein the coupling layer (104, 104', 404, 504, 604) has a percent elongation of no greater than 50%.

12. A kit (101) comprising:
the nasogastric tube securement system (100) of any of the preceding claims, and
a release liner (103),
wherein the base layer (102, 102') and the coupling layer (104, 104', 404, 504, 604) of the nasogastric tube securement system (100) are provided together on the release liner (103).

13. The kit of claim 12, further comprising:
a first tape strip (106) provided on the release liner (103), the first tape strip (106) comprising a securing adhesive (107) and configured to be wrapped about at least a portion of a circumference of the nasogastric tube; and
a second tape strip (108) provided on the release liner (103), the second tape strip (108) comprising a skin-contact adhesive (109) and configured to be adhered to another portion of the subject's skin.

## Patentansprüche

1. System zur Sicherung einer transnasalen Magensonde (100), wobei das System aufweist:
eine Basisschicht (102, 102'), die dafür konfiguriert ist, um an eine Nase angehaftet zu werden, wobei die Basisschicht (102, 102') eine erste Hauptoberfläche (110, 110'), die einen Hautkontaktkleber (112, 112') aufweist, und eine zweite Hauptoberfläche (114, 114') gegenüber der ersten Hauptoberfläche (110, 110') hat, wobei die Basisschicht (102, 102') einen Fussabdruckbereich von A aufweist; und
eine Kopplungsschicht (104, 104', 404, 504, 604), die aufweist:
ein erstes Ende (120, 120', 420, 520, 620), das Kopplungsmittel aufweist, die dafür konfiguriert sind, um repositionierbar an die zweite Hauptoberfläche (114, 114') der Basisschicht (102, 102') gekoppelt zu werden, wobei das erste Ende (120, 120') einen Fussabdruckbereich von mindestens 0,3 A aufweist, wobei eine Breite des ersten Endes (120, 120', 420, 520, 620) der Kopplungsschicht (104, 104', 505, 604) eine Form aufweist, die dafür geeignet ist, einen Nasenrücken und mindestens einen Teil der Nasenseitenflächen abzudecken,
ein zweites Ende (122, 122', 422, 522, 622), das dafür konfiguriert ist, um eine transnasale Magensonde (T) zu sichern, und
einen linearen mittleren Abschnitt (124, 424, 524, 624), der das erste Ende (120, 120', 420, 520, 620) und das zweite Ende (122, 122', 422, 522, 622) verbindet, wobei der mittlere Abschnitt (124, 424, 524, 624) sich entlang einer Längsrichtung (D) erstreckt,
wobei das erste Ende (120, 120', 420, 520, 620) und das zweite Ende (122, 122', 422, 522, 622) jeweils breiter sind als der mittlere Abschnitt (124, 424, 524, 624) in der seitlichen Richtung (L), die im Wesentlichen senkrecht in Bezug auf die Längsrichtung (D) ausgerichtet ist, wobei mindestens der mittlere Abschnitt (124, 424, 524, 624) und das zweite Ende (122, 122', 422, 522, 622) der Kopplungsschicht (104, 104', 404, 504, 604) im Allgemeinen eine "L"-Form bilden und wobei die Kopplungsschicht (104, 104', 404, 504, 604) um die Längsrichtung (D) asymmetrisch ist.

2. System nach Anspruch 1, wobei das zweite Ende (122, 122', 422, 522, 622) der Kopplungsschicht (104, 104', 404, 504, 604) in der seitlichen Richtung (L) breiter ist als das erste Ende (120, 120', 420, 520, 620).

3. System nach Anspruch 1 oder 2, wobei die Kopplungsschicht (104, 104', 604) aufweist:
eine erste Hauptoberfläche (130, 130', 630), die dafür konfiguriert ist, um mit der zweiten Hauptoberfläche (114, 114') der Basisschicht (102, 102') gekoppelt zu werden, wobei die erste Hauptoberfläche (130, 130', 630) einen Kleber (132, 132') aufweist, und
eine zweite Hauptoberfläche (134, 134') gegenüber der ersten Hauptoberfläche (130, 130', 630), wobei die zweite Hauptoberfläche (134, 134') von mindestens einem Abschnitt der Kopplungsschicht (104, 104', 604) ein Trennmittel für den Kleber aufweist (132, 132').

4. System nach einem der Ansprüche 1-3, wobei die Kopplungsschicht (104, 104', 604) eine erste Hauptoberfläche (130, 130', 630) aufweist, wobei die Kopplungsschicht (104, 104', 604) ferner einen Kleber (132, 132') auf der ersten Hauptoberfläche (130, 130', 630) aufweist und wobei die zweite Hauptoberfläche (114, 114') der Basisschicht (102, 102') ein Trennmittel (136) für den Kleber (132, 132') auf der ersten Hauptoberfläche (130, 130', 630) der Kopplungsschicht (104, 104', 604) aufweist.

5. System nach einem der Ansprüche 1-4, wobei die Kopplungsschicht (104') eine erste Hauptoberfläche (130') aufweist, wobei die Kopplungsschicht (104') eine erste Berührungsoberfläche (142') eines mechanischen Befestigungselements (143') auf der ersten Hauptoberfläche (130') aufweist und wobei die zweite Hauptoberfläche (114') der Basisschicht (102') eine zweite Berührungsoberfläche (144') des mechanischen Befestigungselements (143') aufweist, das dafür konfiguriert ist, um mit der ersten Berührungsoberfläche (143') in Eingriff zu kommen.

6. System nach einem der Ansprüche 1-5, wobei das erste Ende (120, 120', 420, 520, 620) und das zweite Ende (122, 122', 422, 522, 622) der Kopplungsschicht (104, 104', 404, 504, 604) jeweils in Bezug auf den mittleren Abschnitt (124, 424, 524, 624) der Kopplungsschicht (104, 104', 404, 504, 604) seitlich zentriert sind.

7. System nach einem der Ansprüche 1-6, wobei der mittlere Abschnitt (124, 424, 524, 624) der Kopplungsschicht (122, 122', 422, 522, 622) eine einheitliche Breite aufweist.

8. System nach einem der Ansprüche 1-7, wobei das erste Ende (120, 120', 420, 520, 620) der Kopplungsschicht (104, 104', 404, 504, 604) einen Fußabdruckbereich von mindestens 0,5 A aufweist.

9. System nach einem der Ansprüche 1-8, wobei das erste Ende (120, 120', 420, 520, 620) der Kopplungsschicht (104, 104', 404, 504, 604) eine Form aufweist, die die Form der Basisschicht (102, 102') nachahmt.

10. System nach einem der Ansprüche 1-9, wobei das zweite Ende (122, 122', 422, 522, 622) der Kopplungsschicht (104, 104', 404, 504, 604) eine erste Hauptoberfläche (130, 130', 630) aufweist, die einen Sicherungskleber (132, 132') derart aufweist, dass der Sicherungskleber (132, 132') auf dem ersten seitlichen Abschnitt (655) positioniert ist, um an dem Sicherungskleber (132, 132') auf dem zweiten seitlichen Abschnitt (657) anzuhaften, wenn der erste seitliche Abschnitt (655) und der zweite seitliche Abschnitt (657) in einer überlappenden Beziehung stehen.

11. System nach einem der Ansprüche 1-10, wobei die Basisschicht (102, 102') eine prozentuale Dehnung von mindestens 200 % aufweist und wobei die Kopplungsschicht (104, 104', 404, 504, 604) eine prozentuale Dehnung von nicht mehr als 50 % aufweist.

12. Kit (101), das aufweist:
das System zur Sicherung einer transnasalen Magensonde (100) nach einem der vorstehenden Ansprüche, und
eine Trennlage (103);
wobei die Basisschicht (102, 102') und die Kopplungsschicht (104, 104', 404, 504, 604) des Systems zur Sicherung einer transnasalen Magensonde (100) zusammen auf der Trennlage (103) bereitgestellt sind.

13. Kit nach Anspruch 12, ferner aufweisend:
einen ersten Bandstreifen (106), der auf der Trennlage (103) bereitgestellt ist, wobei der erste Bandstreifen (106) einen Sicherungskleber (107) aufweist und dafür konfiguriert ist, um mindestens um einen Abschnitt eines Umfangs der transnasalen Magensonde gewickelt zu werden; und
einen zweiten Bandstreifen (108), der auf der Trennlage (103) bereitgestellt ist, wobei der zweite Bandstreifen (108) einen Hautkontaktkleber (109) aufweist und dafür konfiguriert ist, um an einen anderen Abschnitt der Haut des Subjekts anzuhaften.

## Revendications

1. Système de fixation de tube nasogastrique (100), le système comprenant :
une couche de base (102, 102') configurée pour être adhérée à un nez, la couche de base (102, 102') ayant une première surface principale (110, 110') comprenant un adhésif de contact cutané (112, 112') et une deuxième surface principale (114, 114') opposée à la première surface principale (110, 110'), la couche de base (102, 102') ayant une aire d'encombrement de A ; et
une couche de couplage (104, 104', 404, 504, 604) comprenant
une première extrémité (120, 120', 420, 520, 620) comprenant des moyens de couplage configurés pour être couplés de manière repositionnable à la deuxième surface principale (114, 114') de la couche de base (102, 102'), la première extrémité (120, 120') ayant une aire d'encombrement d'au moins 0,3 A, dans lequel une largeur de la première extrémité (120, 120', 420, 520, 620) de la couche de couplage (104, 104', 505, 604) a une forme appropriée pour recouvrir un pont nasal et au moins une partie des côtés latéraux du nez,
une deuxième extrémité (122, 122', 422, 522, 622) configurée pour fixer un tube nasogastrique (T), et
une section médiane linéaire (124, 424, 524, 624) connectant la première extrémité (120, 120', 420, 520, 620) et la deuxième extrémité (122, 122', 422, 522, 622), la section médiane (124, 424, 524, 624) allongée le long d'une direction longitudinale (D),
dans lequel la première extrémité (120, 120', 420, 520, 620) et la deuxième extrémité (122, 122', 422, 522, 622) sont chacune plus larges que la section médiane (124, 424, 524, 624) dans la direction latérale (L) qui est orientée sensiblement de manière perpendiculaire par rapport à la direction longitudinale (D), dans lequel au moins la section médiane (124, 424, 524, 624) et la deuxième extrémité (122, 122', 422, 522, 622) de la couche de couplage (104, 104', 404, 504, 604) forment généralement une forme en "L", et dans lequel la couche de couplage (104, 104', 404, 504, 604) est asymétrique autour de la direction longitudinale (D).

2. Système selon la revendication 1, dans lequel la deuxième extrémité (122, 122', 422, 522, 622) de la couche de couplage (104, 104', 404, 504, 604) est plus large dans la direction latérale (L) que la première extrémité (120, 120', 420, 520, 620).

3. Système selon la revendication 1 ou 2, dans lequel la couche de couplage (104, 104', 604) inclut
une première surface principale (130, 130', 630) configurée pour être couplée à la deuxième surface principale (114, 114') de la couche de base (102, 102'), la première surface principale (130, 130', 630) comprenant un adhésif (132, 132'), et
une deuxième surface principale (134, 134') opposée à la première surface principale (130, 130', 630), dans lequel la deuxième surface principale (134, 134') d'au moins une partie de la couche de couplage (104, 104', 604) inclut un agent de libération pour l'adhésif (132, 132').

4. Système selon l'une quelconque des revendications 1 à 3, dans lequel la couche de couplage (104, 104', 604) inclut une première surface principale (130, 130', 630), dans lequel la couche de couplage (104, 104', 604) inclut en outre un adhésif (132, 132') sur la première surface principale (130, 130', 630), et dans lequel la deuxième surface principale (114, 114') de la couche de base (102, 102') inclut un agent de libération (136) pour l'adhésif (132, 132') sur la première surface principale (130, 130', 630) de la couche de couplage (104, 104'. 604).

5. Système selon l'une quelconque des revendications 1 à 4, dans lequel la couche de couplage (104') inclut une première surface principale (130'), dans lequel la couche de couplage (104') inclut une première surface de jointement (142') d'une fixation mécanique (143') sur la première surface principale (130'), et dans lequel la deuxième surface principale (114') de la couche de base (102') inclut une deuxième surface de jointement (144') de la fixation mécanique (143') configurée pour venir en prise avec la première surface de jointement (143').

6. Système selon l'une quelconque des revendications 1 à 5, dans lequel la première extrémité (120, 120', 420, 520, 620) et la deuxième extrémité (122, 122', 422, 522, 622) de la couche de couplage (104, 104', 404, 504, 604) sont chacune centrées latéralement par rapport à la section médiane (124, 424, 524, 624) de la couche de couplage (104, 104', 404, 504, 604).

7. Système selon l'une quelconque des revendications 1 à 6, dans lequel la section médiane (124, 424, 524, 624) de la couche de couplage (122, 122', 422, 522, 622) a une largeur uniforme.

8. Système selon l'une quelconque des revendications 1 à 7, dans lequel la première extrémité (120, 120', 420, 520, 620) de la couche de couplage (104, 104', 404, 504, 604) a une aire d'encombrement d'au moins 0,5 A.

9. Système selon l'une quelconque des revendications 1 à 8, dans lequel la première extrémité (120, 120', 420, 520, 620) de la couche de couplage (104, 104', 404, 504, 604) a une forme qui imite la forme de la couche de base (102, 102').

10. Système selon l'une quelconque des revendications 1 à 9, dans lequel la deuxième extrémité (122, 122', 422, 522, 622) de la couche de couplage (104, 104', 404, 504, 604) inclut une première surface principale (130, 130', 630) comprenant un adhésif de fixation (132, 132'), de telle sorte que l'adhésif de fixation (132, 132') sur la première partie latérale (655) est positionné pour adhérer à l'adhésif de fixation (132, 132') sur la deuxième partie latérale (657) lorsque la première partie latérale (655) et la deuxième partie latérale (657) sont dans une relation de chevauchement.

11. Système selon l'une quelconque des revendications 1 à 10, dans lequel la couche de base (102, 102') a un pourcentage d'allongement d'au moins 200 %, et dans lequel la couche de couplage (104, 104', 404, 504, 604) a un pourcentage d'allongement non supérieur à 50 %.

12. Trousse (101) comprenant :
le système de fixation de tube nasogastrique (100) selon l'une quelconque des revendications précédentes, et
une feuille anti-adhésive (103),
dans laquelle la couche de base (102, 102') et la couche de couplage (104, 104', 404, 504, 604) du système de fixation de tube nasogastrique (100) sont fournies ensemble sur la feuille anti-adhésive (103).

13. Trousse selon la revendication 12, comprenant en outre :
une première bande de ruban (106) fournie sur la feuille anti-adhésive (103), la première bande de ruban (106) comprenant un adhésif de fixation (107) et configurée pour être enroulée autour d'au moins une partie d'une circonférence du tube nasogastrique ; et
une deuxième bande de ruban (108) fournie sur la feuille anti-adhésive (103), la deuxième bande de ruban (108) comprenant un adhésif de contact cutané (109) et configurée pour être adhérée à une autre partie de la peau d'un sujet.
